(19)

Europäisches
Patentamt
European
Patent Office
Office européen
des brevets

(11) EP 4 707 843 A1

(12) EUROPEAN PATENT APPLICATION

(43) Date of publication:
11.03.2026 Bulletin 2026/11

(51) International Patent Classification (IPC):
G01R 33/56 (2006.01)   G01R 33/565 (2006.01)
G01R 33/561 (2006.01)   A61B 5/055 (2006.01)
G01R 33/48 (2006.01)

(21) Application number: 24199528.1

(22) Date of filing: 10.09.2024

(52) Cooperative Patent Classification (CPC):
G01R 33/56509; A61B 5/055; G01R 33/5608;
G01R 33/5611; G01R 33/4824

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Koninklijke Philips N.V.
5656 AG Eindhoven (NL)

(72) Inventors:
• DONEVA, Mariya Ivanova
  Eindhoven (NL)
• KEUPP, Jochen
  Eindhoven (NL)

(74) Representative: Philips Intellectual Property &
Standards
High Tech Campus 52
5656 AG Eindhoven (NL)

(54) **IMPROVING IMAGE QUALITY IN MOTION CORRECTED MAGNETIC RESONANCE IMAGING**

(57)    Disclosed herein is an image reconstruction method of reconstructing an augmented magnetic resonance image (310). The method comprises receiving (500) motion corrected k-space data (102) having a first tissue contrast modality; identifying (502) sparse k-space regions (104) within the motion corrected k-space data; receiving (504) an alternative magnetic resonance image (300) with a second tissue contrast modality. The method further comprises generating (506) a synthetic magnetic resonance image (302) which has the first tissue contrast modality in response to inputting the alternative magnetic resonance image into a magnetic resonance modality conversion neural network (312) and generating (508) synthetic k-space data from the synthetic magnetic resonance image. An augmented magnetic resonance image is reconstructed (510) by using the motion corrected k-space data such that the sparse k-space regions of the motion-corrected k-space data are augmented with the synthetic k-space data. Systems are presented that are configured to perform the method.

Fig. 3

**Description**

TECHNICAL FIELD

The invention relates to magnetic resonance imaging, in particular to motion correction in magnetic resonance imaging.

BACKGROUND

A large static magnetic field is used by Magnetic Resonance Imaging (MRI) scanners to align the nuclear spins of atoms as part of the procedure for producing images within the body of a subject. This large static magnetic field is referred to as the B0 field or the main magnetic field. In MRI, the atomic spins are manipulated using gradient magnetic fields and radio frequency (RF) pulses. The measured data is acquired in k-space over several pulse repetitions or acquisitions. However, acquiring a complete set of k-space for reconstructing an image is time consuming and a subject may move during the acquisition, which can cause blurring and other types of image artifacts.

Motion correction is often used in magnetic resonance imaging to reduce artifacts caused by subject movement during the scan. One of the advanced techniques employed for motion correction is the PROPELLER (Periodically Rotated Overlapping ParallEL Lines with Enhanced Reconstruction) method. This technique involves the acquisition of data in a series of overlapping, rotating blades through k-space, rather than traditional Cartesian sampling. The redundancy in these overlapping k-space blades allows for the detection and correction of in-plane motion by aligning these blades relative to one another. During motion correction, the k-space samples can be repositioned or regridded according to the estimated motion parameters, effectively minimizing the motion artifacts. This realignment of k-space data ensures that the final reconstructed image is more accurate, preserving anatomical details.

Besides the PROPELLER method, several other techniques can be used to correct k-space data for motion in MRI. For example, navigator-based motion correction may be used. Additional signals, or "navigators", are periodically acquired to track motion throughout the scan. These navigators can be integrated into the imaging sequence and provide motion estimates that are then used to adjust the k-space data in real-time or retrospectively. Further retrospective motion correction techniques, such as those using image-based registration, align images from different time points by estimating motion parameters post-acquisition and correcting the corresponding k-space data. Self-navigation techniques leverage redundant information in the acquired data itself, to estimate and correct for motion without the need for additional navigator pulses. Finally, motion correction systems employ external devices, like cameras or other motion sensors, to monitor subject movement. This motion information can be used for motion correction in reconstruction as well as in real-time to adjust the imaging parameters or k-space trajectory dynamically during the scan.

Yang, Q., Li, N., Zhao, Z. et al., "MRI Cross-Modality Image-to-Image Translation," Sci Rep 10, 3753 (2020); https://doi.org/10.1038/s41598-020-60520-6, discloses a cross-modality generation framework that learns to generate translated modalities from given modalities in MR images. The method performs Image Modality Translation (abbreviated as IMT) by means of a deep learning model that leverages conditional generative adversarial networks (cGANs). The framework jointly exploits the low-level features (pixel-wise information) and high-level representations (e.g. brain tumors, brain structure like gray matter, etc.) between cross modalities, which are important for resolving the challenging complexity in brain structures.

SUMMARY

The invention provides an image reconstruction method, a computer program, a medical system, and a data structure in the independent claims. Embodiments are given in the dependent claims.

In one aspect an image reconstruction method of reconstructing an augmented magnetic resonance image is disclosed. The method comprises receiving motion-corrected k-space data descriptive of a field of view of a subject. The motion-corrected k-space data has a first tissue contrast modality. The method further comprises identifying sparse k-space regions within the motion-corrected k-space data. The method further comprises receiving an alternative magnetic resonance image descriptive of the field of view of the subject.

The alternative magnetic resonance image has a second tissue contrast modality. The method further comprises receiving a synthetic magnetic resonance image which has the first tissue contrast modality in response to inputting the alternative magnetic resonance image into a magnetic resonance modality conversion neural network. The method further comprises generating synthetic k-space data from the synthetic magnetic resonance image. The method further comprises reconstructing the augmented magnetic resonance image using the motion-corrected k-space data such that the sparse k-space regions of the motion-corrected k-space data are augmented with the synthetic k-space data. The final augmented k-space data is transformed to image space for the first tissue contrast modality (e.g. via a compressed sensing reconstruction) may provide improved image quality as compared to images created from the initial motion-corrected k-space data.

In another aspect, a computer program comprising machine-executable instructions is disclosed. Execution of the machine-executable instructions causes a computational system to perform the image reconstruction method.

In another aspect, a medical system is disclosed. The medical system comprises a memory storing machine-executable instructions and a computational system. The execution of the machine-executable instructions causes the computational system to receive motion-corrected k-space data descriptive of a field of view of a subject. The motion-corrected k-space data has a first tissue contrast modality. Execution of the machine-executable instructions further causes the computational system to identify sparse k-space regions within the motion-corrected k-space data. Execution of the machine-executable instructions further causes the computational system to receive an alternative magnetic resonance image descriptive of the field of view of the subject. The alternative magnetic resonance image has a second tissue contrast modality. Execution of the machine-executable instructions further causes the computational system to receive a synthetic magnetic resonance image which has the first tissue contrast modality in response to inputting the alternative magnetic resonance image into a magnetic resonance modality conversion neural network. Execution of the machine-executable instructions further causes the computational system to generate synthetic k-space data from the synthetic magnetic resonance image. Execution of the machine-executable instructions further causes the computational system to reconstruct the augmented magnetic resonance image using the motion-corrected k-space data such that the sparse k-space regions of the motion-corrected k-space data are augmented with the synthetic k-space data.

In another aspect a data structure is disclosed. The data structure comprises motion-corrected k-space data descriptive of a field of view of a subject and having a first tissue contrast modality. The data structure further comprises synthetic k-space data also descriptive of the field of view of the subject and having a second tissue contrast modality. The data structure further comprises an identification of sparse k-space regions within the motion-corrected k-space data.

BRIEF DESCRIPTION OF THE DRAWINGS

In the following preferred embodiments of the invention will be described, by way of example only, and with reference to the drawings in which:

Fig. 1 illustrates an example of acquired and motion corrected k-space data.
Fig. 2 illustrates sampling patterns of acquired and motion corrected k-space data.
Fig. 3 illustrates a method of generating an augmented magnetic resonance image.
Fig. 4 illustrates an example of a medical system.
Fig. 5 shows a flow chart illustrating a method of operating a medical system.
Fig. 6 illustrates a further example of a medical system.
Fig. 7 illustrates a further example of a medical system that incorporates a cloud based computing system.

DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

Like numbered elements in these figures are either equivalent elements or perform the same function. Elements which have been discussed previously will not necessarily be discussed in later figures if the function is equivalent.

In an example, there is an image reconstruction method of reconstructing an augmented magnetic resonance image. The method comprises receiving motion-corrected k-space data that is descriptive of a field of view of a subject. The acquired motion-corrected k-space data has a first tissue contrast modality. In magnetic resonance imaging, during the execution of a magnetic resonance imaging protocol k-space data is acquired. This k-space data may then be later Fourier transformed or processed with an iterative compressed sensing algorithm to produce a magnetic resonance image. The tissue contrast modality refers to the parameters in the pulse sequence that were used to acquire the k-space data. The magnetic resonance image can be weighted to show different properties such as proton density, T1 relaxation time, T2 relaxation time and a variety of other parameters. The modality of a particular magnetic resonance image is determined by the acquisition parameters used to acquire the k-space data. Saying that the motion-corrected k-space data has a first tissue contrast modality refers to using a first set of parameters or a first pulse sequence to acquire the k-space data.

The method further comprises identifying sparse k-space regions within the motion-corrected k-space data. At various locations from the origin of the k-space data it may be desired to have a particular distribution of k-space data within a particular neighborhood. If there is less k-space data within a neighborhood then this may result in image artifacts, enhanced noise or other types of degradations in the quality of a magnetic resonance image. For example, for the motion-corrected k-space data, a particular pulse sequence may have been used to acquire this. However, due to subject motion the k-space data becomes inconsistent and needs to be shifted in position or phase during reconstruction to avoid motion artifacts. For example, it may be desired to have a particular k-space sampling pattern but in practice the measurements may be shifted to a different location after motion correction. The sparse k-space regions may encompass a region where the k-space data has fewer samples than below a particular threshold. This threshold may for example be dependent on the origin of k-space and may decrease as the distance from the origin increases.

The method further comprises receiving an alternative magnetic resonance image descriptive of the field of view of the subject. The alternative magnetic resonance image has a second tissue contrast modality. The alternative magnetic resonance image has some redundancy with the motion-corrected k-space data as they are acquired for the same field of view of the subject. However, since the tissue contrast modality is different, the resulting magnetic resonance images may have a different appearance or partly contain different information. The method further comprises receiving a synthetic magnetic resonance image which has the first tissue contrast modality in response to inputting the alternative magnetic resonance image into a magnetic resonance modality conversion neural network. In this step, the synthetic magnetic resonance image is input into a neural network and a synthetic magnetic resonance image is output, which is used to simulate a magnetic resonance image having the first tissue contrast modality. This typically works relatively well because the different tissue contrast modalities often still depict the same anatomical structures. For example, the magnetic resonance modality contrast neural network could be a neural network which is configured for receiving an image and outputting an image. For example, a convolutional neural network, such as a U-net would be a good choice. It could be trained by having pairs of images with the second contrast modality and the first contrast modality that were acquired for the same field of view for subjects.

As an alternative to U-nets a generative adversarial networks (GANs) may be used to simulate an MRI with a first tissue contrast modality using an MRI image with a second tissue contrast modality. One process may use a CNN or a GAN to learn the complex mapping between the input image (e.g., MRI image with the second tissue contrast modality) and the target image (e.g., simulated MRI image with the first tissue contrast modality). The network may be trained on paired datasets, where each input MRI image corresponds to a ground truth image in the target modality. During training, the network learns to minimize the difference between its output and the ground truth target images, usually through loss functions like mean squared error (MSE) or more advanced perceptual losses. The input to the network is the MRI image, and the output is the simulated image in the desired modality. GANs, in particular, consist of a generator that tries to produce realistic images in the target modality and a discriminator that attempts to distinguish between real and generated images, which together improve the accuracy and realism of the simulated output. Training these networks may use a large dataset, optimization techniques like stochastic gradient descent or Adam, and careful tuning of hyperparameters to ensure convergence and high-quality image generation.

The method further comprises generating synthetic k-space data from the synthetic magnetic resonance image. This may for example be accomplished by doing an inverse Fourier transform of the synthetic magnetic resonance image. The method further comprises reconstructing the augmented magnetic resonance image using the motion-corrected k-space data such that the sparse k-space regions of the motion-corrected k-space data are augmented with the synthetic k-space data. For example, performing motion correction on measured k-space data may result in sparse k-space regions in the motion corrected k-space data. To improve the quality of the augmented magnetic resonance image some of the synthetic k-space data is then used to provide information about k-space that is missing from the motion-corrected k-space data, improving the quality of the final image reconstructed from the augmented k-space data.

This for example may be accomplished by inserting synthetic k-space data into the sparse regions. As an alternative, a more complicated reconstruction may be used which uses both sets of k-space data and uses a weighting factor to emphasize certain portions of the synthetic k-space data.

This example may have the benefit that it may provide for improved image quality while retaining the original anatomical features depicted or encoded in the motion-corrected k-space data.

In another example, the method further comprises reconstructing a preliminary magnetic resonance image from the motion-corrected k-space data. The method further comprises generating a registration by spatially registering the synthetic magnetic resonance image and the preliminary magnetic resonance image to each other. The method further comprises generating a modified magnetic resonance image by transforming the synthetic magnetic resonance image with an elastic deformation, a rigid body transformation, and/or rotations controlled by the registration. The synthetic k-space data is generated from the modified magnetic resonance image. In this example, the quality of the synthetic k-space data may be improved by performing registration so that the two images are aligned. In some instances, if the acquisition is performed at the same time, it may not be necessary or beneficial to perform such a registration. However, this may be used to ensure that the synthetic k-space data matches the motion-corrected k-space data as well as possible.

In another example, the sparse k-space regions are augmented by inserting the synthetic k-space data from the sparse k-space regions into the measured k-space data. In this example the sparse k-space regions of the measured k-space data are supplemented by inserting or pasting k-space data from the synthetic k-space data. This may provide an extremely simple means numerically to augment the measured k-space data with the synthetic k-space data.

In another example, the augmented magnetic resonance image is reconstructed using an optimization. The optimization may for example be configured for performing a parallel imaging and/or compressed sensing reconstruction. This may be beneficial as it may provide for a means of reconstructing the augmented magnetic resonance image with a reduced amount of k-space data.

As an example of an image reconstruction optimization, let p represent the motion free MRI image to be reconstructed, and m be the corresponding motion corrupted k-space data. The operator **T(t)** describes the (continuous) motion for the duration of the scan. This is the forward motion operator that transforms the motion free image to a motion corrupted image. For practical considerations, we assume that the acquired data can be split in N segments corresponding to N motion states, which describe the motion effect on the voxel locations of the motion free image. Then we can define **T$_n$** (n = 1... N) as the transformation from the motion free image to the motion state n. **T$_n$** describes either rigid or non-rigid body motion.

In a typical embodiment for rigid body motion in three dimensions, each **T$_n$** is represented by a set of 6 parameters (3 rotations, 3 translations). Here it is assumed - as an approximation - that motion happens in between the segments and the motion state is stationary within each segment. For an extension to non-rigid motion, a motion vector field may be parametrized by a limited set of parameters, e.g. by describing weights for deformation basis functions for each motion state. As a further extension, a motion state may also describe a uniform, or uniformly accelerated state of motion, during the duration of one or more segments **T$_n$**, using a limited set of parameters. One example would be a constant rotation during head motion or uniform stretching of tissue during an inhalation.

Then the reconstruction of a motion corrected image can be formulated as the following optimization problem:

$$p \ = \ argmin \sum_n \|F(M_n)ST_n p - m_n\|_2$$

Here S are the coil sensitivity maps, $F(M_n)$ is the Fourier transform (which can be Cartesian or non-Cartesian) computed at k-space coordinates $M_n$ describing the prescribed sampling pattern for the n$^{th}$ segment. Combining the k-space coordinates $M_n$ for all segments leads to the total prescribed sampling pattern M.

Usually, undersampling is used to accelerate the scan, which requires adding regularization to the reconstruction. This may be part of a compressed sensing reconstruction. This can be a combination of L1 and L2 regularization terms as shown in the equation below:

$$p \ = \ argmin \sum_n \|F(M_n)ST_n p - m_n\|_2 \ + \lambda_1 \| Rp \|_2 \ + \lambda_2 \| \Psi p \|_1$$

The sparsifying transform $\Psi$ can be either a fixed transform like finite differences or wavelets, or a learned transform.

The motion transform **T$_n$** may not exactly represent the motion, which may lead to data inconsistencies and residual motion artifacts. However, even with a perfect knowledge of the motion, the reconstruction problem with motion is often worse conditioned compared to the reconstruction problem without motion. An explanation for this is that the motion, in particular the rotation part of the motion, results in a modified effective sampling pattern that may have larger gaps leading to worse conditioning.

To improve the conditioning of the reconstruction problem one may use a prior that effectively aims to fill the gaps in k-space. This can be achieved by including an additional regularization term in k-space based on the effective sampling pattern and an estimation of the image based on image translation. Here by image translation one means estimating an image with a given tissue contrast modality from an image with a different tissue contrast modality.

Image (contrast) translation is used to estimate a synthetic image **q** with the same contrast as the motion corrupted image from one or more of the scans that were performed in the same exam. The image **q** is estimated as a magnitude image in this setting.

This can be done using different architectures including U-net, Resnet, diffusion models, vision transformers, among others.

Since the motion correction can be done using an arbitrary reference motion state, the motion state of the synthetic image **q** can be used as a reference and the motion parameter estimation and correction is done with respect to that reference. A specific final static motion state for the corrected image reconstruction may be requested, like a specific head positioning in the field of view (FOV) or positioning as seen in the survey/localizer. Then, the scan used to create the synthetic image, or the synthetic image itself, can be spatially co-registered for this state. It is also possible to derive a synthetic image **q** from a set of different acquisitions of the same patient, which then also need to be co-registered to one another for a consistent reconstruction.

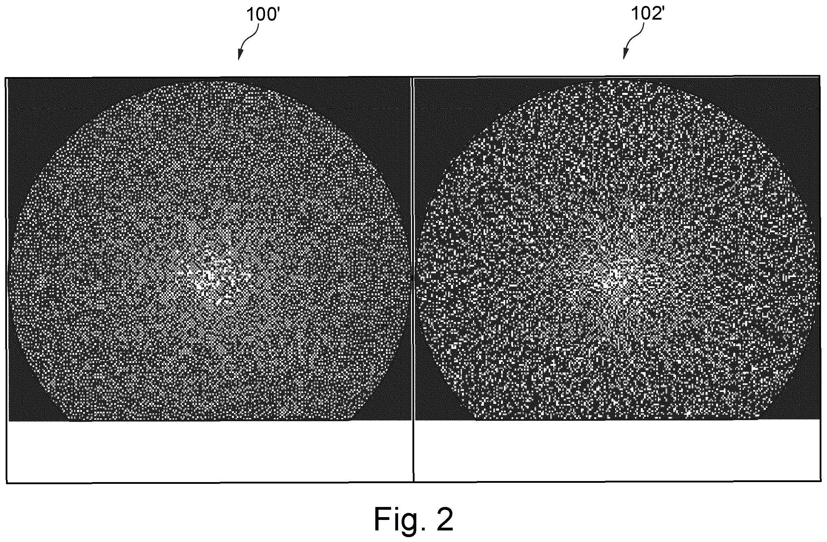

With the estimated motion one can compute an effective sampling pattern **M'** from the original sampling pattern **M. M'** may be obtained by applying the motion transforms **T$_n$** to original sampling pattern segment by segment and recombining all segments for the effective sampling pattern. An example is shown in Figure 2 for a 3D Cartesian sampling pattern. This can be applied for all kinds cartesian or non-cartesian (radial, spiral, propeller etc.) k-space sampling patterns.

In another example, the optimization comprises an additional regularization term that incorporates the synthetic k-space data. The regularization term allows augmentation of the measured k-space data with synthetic k-space data, without directly inserting it into the original motion-corrected k-space data.

Continuing the example above and extending it with a regularization term, the synthetic image **q** together with the effective sampling pattern **M'** can be used to improve the reconstruction by including them in a regularization term in the image reconstruction problem.

$$p = argmin \sum_n \|F(M_n)ST_n p - m_n\|_2 + \lambda_1 \| Rp\|_2 + \lambda_2 \| \Psi p\|_1 + \lambda_3 \|WFS(qe^{i\varphi} - p)\|_2$$

Here F is the Fourier transform for a fully sampled k-space and the weights W select the k-space locations for which we are going to use the synthetic image q as a prior. W is a function of the effective and prescribed sampling patterns

$$W = f(M, M')$$

In the extreme case, it is an indicator function which is 1 for locations that are sampled by M and not by M', and 0 otherwise. Alternatively, a smooth transition between 1 and 0 can be defined using the prior also for neighboring k-space locations with a reduced weight.

The phase term $e^{i\varphi}$ is taken from the current estimation of the image **p,** ensuring that **q** and **p** have the same phase. Typical image contrast translations do not provide useful phase information for the synthetic image **q** but complex images are required in the iterative reconstruction. The phase term is newly estimated and updated in each iteration.

In another example, the additional regularization term comprises weighting factors that are configured for augmenting the sparse k-space regions with the synthetic k-space data. For example, in the full optimization problem the full dataset for the motion-corrected k-space data and the full dataset for the synthetic k-space data may both be included. Then, on top of this, the weighting factors are used to select which data points or regions of the synthetic k-space data contribute to the reconstruction of the augmented magnetic resonance image and also by how much. This may provide for an improved means of augmenting the magnetic resonance image.

In another example, the additional regularization term is configured to use the synthetic magnetic resonance image as a prior that constrains the optimization. This for example may be beneficial if the synthetic magnetic resonance image has fewer motion artifacts than the motion-corrected k-space data.

In another example, the optimization is a joint optimization further configured for performing motion correction. For example, the step of receiving the motion-corrected k-space data may be performed as the optimization problem is solved and may be repeated multiple times. For example, the selection of which regions of the motion-corrected k-space data are sparse could be determined on the fly and then supplemented during each iteration of the joint optimization. In the above example, the motion parameters **T$_n$** are known or are measured ahead of time. In the optimization problem, the motion parameters **T$_n$** could instead be initial motion parameters and the overall optimization could be extended by varying the motion parameters **T$_n$**.

In another example, the sparse k-space regions are repeatedly identified during the joint optimization. For example, as part of the iterations of the joint optimization, the k-space data may be searched for sparse regions. The sparse k-space regions of the motion-corrected k-space data may then be augmented with the synthetic k-space data repeatedly and are also adjusted during the joint optimization. For example, during one iteration of the joint optimization there may be a trial of a particular set of motion parameters to adjust the position of the motion-corrected k-space data. This will then lead to a determination of which are the sparse regions and then for each loop of the optimization the contribution of the synthetic k-space data can be adjusted.

In another example, the sparse k-space region is determined by searching the measured k-space data for regions below a predetermined k-space sampling density. For example, a neighborhood may be moved around the measured k-space data and it can be determined how many sample points are within this neighborhood. For a particular size, and also in some instances depending on the distance from the origin, there may be a threshold value of the number of k-space samples within a neighborhood. This may be a very effective means of determining the sparse k-space regions without needing to keep track of the sampled data points during the pulse sequence.

In another example, the image reconstruction method further comprises controlling a magnetic resonance imaging system. The magnetic resonance imaging system comprises a subject motion detection component configured for measuring motion data descriptive of subject motion. The subject motion detection component may be a system or device for tracking subject motion or it may also be a software component. For example, the motion parameters could be detected using navigator data, self-navigation such as the propeller technique, or also using motion parameters determined using a joint optimization. The method may further comprise acquiring the k-space data as k-space data portions. These may for example be individual acquisitions of k-space data. The method further comprises acquiring motion data for the k-space data portions using the subject motion detection component and for example, the k-space data portions may be labeled with a particular motion data or parameter. The method further comprises constructing the motion-corrected k-space data using the measured k-space data and the motion data.

In another example, the first pulse sequence commands are configured to acquire the measured k-space data according to a k-space sampling pattern. The sparse k-space regions are at least partially determined using the k-space sampling pattern and the motion data. When the sampling pattern is known and the motion data is known it may be possible to predict the position of the k-space samples.

In another example, the subject motion detection component is any one of the following: a software component configured for driving the motion data for a magnetic resonance navigator data, a software component configured to implement a joint optimization image reconstruction algorithm, a respiratory belt, or a camera system. These may be used separately or together to determine the motion data.

In another example, the method further comprises controlling the magnetic resonance imaging system with second pulse sequence commands to acquire alternative k-space data. The method further comprises acquiring the alternative k-space data by controlling the magnetic resonance imaging system with the second pulse sequence commands and then reconstructing the alternative magnetic resonance image from the alternative k-space data.

In another example, there is a computer program comprising machine-executable instructions. Execution of the machine-executable instructions causes a computational system to perform an example of the image reconstruction method.

In a further example, there is a medical system. The medical system comprises a memory storing machine-executable instructions and a computational system. Execution of the machine-executable instructions causes the computational system to receive motion-corrected k-space data descriptive of a field of view of a subject. The motion-corrected k-space data has a first contrast modality. Execution of the machine-executable instructions further causes the computational system to identify sparse k-space regions within the motion-corrected k-space data. Execution of the machine-executable instructions further causes the computational system to receive an alternative magnetic resonance image that is descriptive of the field of view of the subject. The alternative magnetic resonance image has a second tissue contrast modality. Execution of the machine-executable instructions further causes the computational system to receive a synthetic magnetic resonance image which has the first tissue contrast modality in response to inputting the alternative magnetic resonance image into a magnetic resonance modality conversion neural network.

Execution of the machine-executable instructions further causes the computational system to generate synthetic k-space data from the synthetic magnetic resonance image. Execution of the machine-executable instructions further causes the computational system to reconstruct the augmented magnetic resonance image using the motion-corrected k-space data such that the sparse k-space regions of the motion-corrected k-space data are augmented with the synthetic k-space data.

In a further example, there is a data structure. The data structure comprises motion-corrected k-space data. The motion-corrected k-space data is descriptive of a field of view of a subject and has a first tissue contrast modality. The data structure further comprises a synthetic k-space data that is also descriptive of the field of view of the subject. The synthetic k-space data also has the first tissue contrast modality. The data structure further comprises identification of sparse k-space regions within the motion-corrected k-space data. The data structure may be beneficial because it may provide for a means of providing for an augmented magnetic resonance image that has improved image quality over what would be possible from just constructing a magnetic resonance image from the motion-corrected k-space data alone.

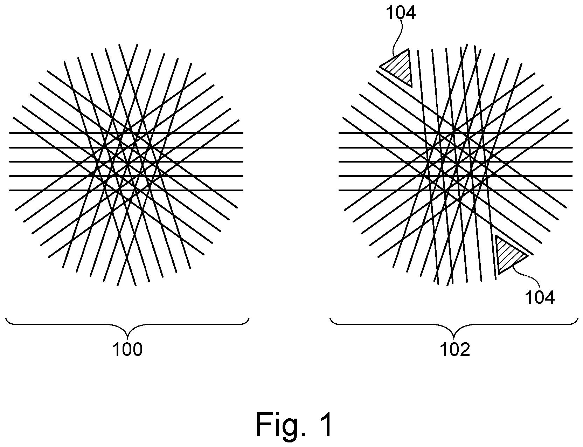

Fig. 1 shows an example of acquired k-space data 100. In this example, the acquired k-space data 100 was acquired using a so-called propeller technique. In propeller magnetic resonance imaging individual blades of k-space data can be reconstructed into low resolution images and then used to perform motion correction. In this same figure there is then motion-corrected k-space data 102 that was corrected using this propeller technique. The position of the blades of k-space data have been shifted resulting in two regions where there is worse coverage of k-space data by sample points. These are labeled 104 and are examples of sparse k-space regions. Examples, as described herein, may be used to implement the region of k-space 104 which are sparse as is illustrated in Fig. 1.

Fig. 2 illustrates a further example of measured k-space data 100'. In this example, a $k_y$, $k_z$ sampling pattern from a three-dimensional Cartesian compressed sensing acquisition with $k_x$ being a linear readout is shown. Sampling pattern 100' is the original sampling pattern. After motion correction has been performed on the measured k-space data 100', this results in the motion-corrected k-space data 102'. In the motion-corrected k-space data 102' it can be seen that some areas show enhanced sampling density while there are patchy areas with very low sampling density. Examples, as described herein, may be used to supplement these patchy areas.

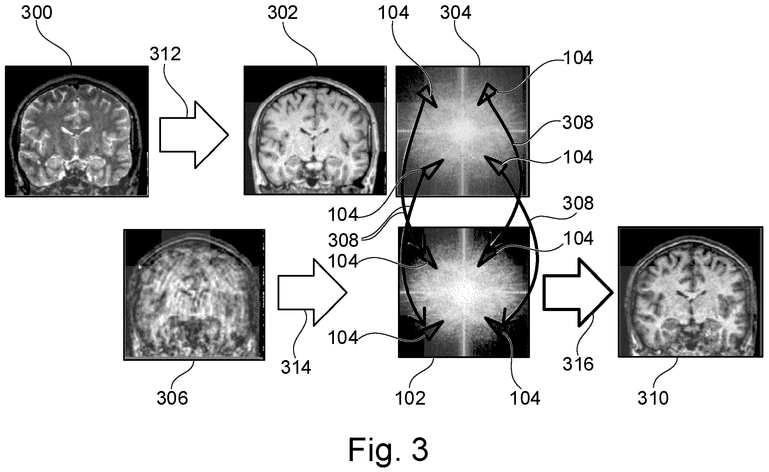

Fig. 3 illustrates a method of generating an augmented magnetic resonance image 310. At the top there is an alternative magnetic resonance image 300 that is descriptive of a field of view of a subject. The magnetic resonance image 300 has a second tissue contrast modality. This is then converted using a modality conversion neural network 312 to produce a synthetic magnetic resonance image 302. The synthetic magnetic resonance image 302 shows the same view as the alternative magnetic resonance image 300 but it has been converted to a first tissue contrast modality. The synthetic magnetic resonance image 302 may then be inverse Fourier transformed to produce synthetic k-space data 304. Below this is shown a motion-corrected image 306 that was reconstructed from measured k-space data 306. A motion correction 314 operation is performed on the measured k-space data to provide the motion-corrected k-space data 102. Within the motion-corrected k-space data 102 is identified a number of sparse k-space regions 104. These same regions are also identified in the synthetic k-space data 304. An insertion operation 308 is performed to insert synthetic k-space data 304 from the sparse k-space regions 104 into the sparse k-space regions 104 of the motion-corrected k-space data 102. The image reconstruction 316 is then performed on the motion-corrected k-space data 102 that has been augmented with k-space data from the synthetic magnetic resonance image 302 to produce the augmented magnetic resonance image 310. In this example, there was an insertion operation performed and k-space data was explicitly copied or inserted into the motion-corrected k-space data 102 before reconstruction. As an alternative, an optimization problem could also be set up which is used to generate the augmented magnetic resonance image 310 from both the motion-corrected k-space data 102 and the synthetic k-space data 304.

In alternative embodiment and additional magnetic resonance modality conversion neural network can be inserted or added to the image reconstruction 316, so that the augmented magnetic resonance image 310 is with a different tissue contrast modality than the motion-corrected image 306 and the synthetic magnetic resonance image 302.

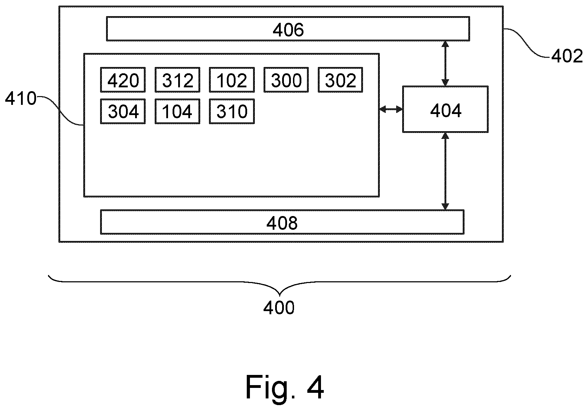

Fig. 4 illustrates an example of a medical system 400. In this example, the medical system 400 comprises a computer 402. The computer 402 may represent one or more computers or computational systems located at one or more locations. The computer 402 comprises a computational system 404 that comprises one or more computational or computing cores. The computational system 404 is in communication with an optional hardware interface 406 and an optional user interface or display 408. The hardware interface 406, if it is present, may for example be used for controlling other components of the medical system 400 such as a magnetic resonance imaging system. The user interface 408, e.g., display, may be used by an operator to control the operation and function of the medical system 400.

The computational system 404 is shown as further being in communication with a memory 410. The memory 410 represents various types of memories that may be used to provide data or commands to the computational system 404. In some examples, the memory 410 is or comprises a non-transitory storage medium. The memory 410 is shown as storing machine-executable instructions 420. The machine-executable instructions 420 enable the computational system 404 to perform such tasks as controlling or communicating with other components of the medical system 400 as well as being able to perform numerical and image processing tasks such as reconstructing magnetic resonance images. The memory 410 is further shown as storing the magnetic resonance modality conversion neural network which is configured for receiving magnetic resonance images with a second tissue contrast modality and then generating a synthetic magnetic resonance image with the first contrast modality. The memory 410 is further shown as storing a motion-corrected k-space data 102. The memory 410 is further shown as storing an alternative magnetic resonance image 300. The alternative magnetic resonance image 300 and the motion-corrected k-space data are descriptive of the same field of view of a subject.

The memory 410 is further shown as containing a synthetic magnetic resonance image 302 that was generated by inputting the alternative magnetic resonance image 300 into the magnetic resonance modality conversion neural network 312. The memory 410 is further shown as containing synthetic k-space data 304 that was generated from the synthetic magnetic resonance image 302. The memory 410 is further shown as containing identification of sparse k-space regions 104 which are located within the motion-corrected k-space data 102. The memory 410 is further shown as containing an augmented magnetic resonance image 310 that was reconstructed using the motion-corrected k-space data 102, the synthetic k-space data 304, and a knowledge of the location of the sparse k-space regions 104. For example, the sparse k-space regions 104 may be supplemented by inserting k-space data from the synthetic k-space data 304.

In examples with motion corrected reconstructions, a motion trajectory can be estimated from the data itself (e.g., by iterative reconstruction of image and trajectory data, by rigid body or elastic registration of sub-images or by navigator information) or obtained through external sensors like a camera. In the following, it is assumed that the motion trajectory is already known.

In motion corrected reconstruction, motion trajectories are used to correct the k-space data by applying transformation operators to the k-space data. If the k-space data is subjected to transformation operators, the k-space structure is altered and the k-space sampling density in relation to motion effects can be estimated from the trajectory data and the k-space sampling scheme used in the MR image acquisition.

Depending on the reconstruction approach, the projection to k-space of the synthetic images can be done either using a Fourier transform (in general on a non-Cartesian grid) or first multiplying by the coil sensitivities to generate multi-coil data.

Inserting the projected k-space data can in some examples be performed by replacing k-space data points or by applying weights for a smooth transition between the original and synthetic k-space parts within the gaps, thus forming a hybrid k-space.

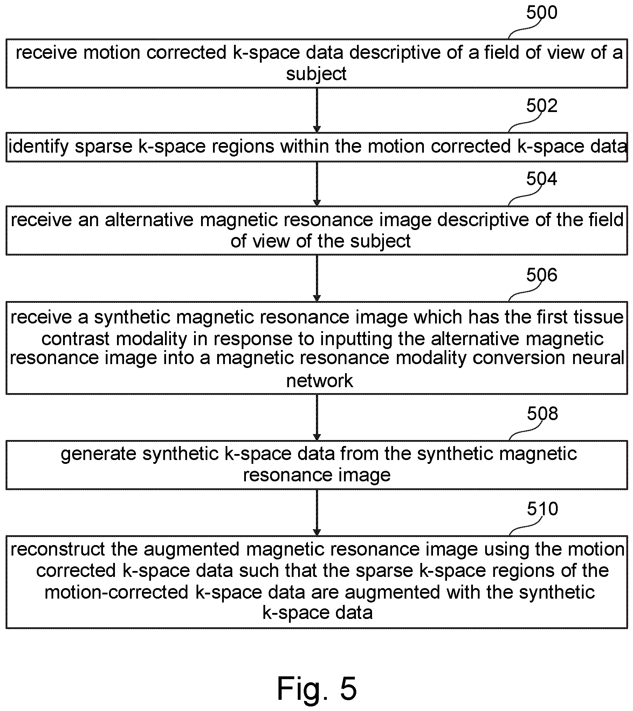

Fig. 5 shows a flowchart which illustrates a method of operating the medical system 400 of Fig. 4. In step 500, the motion-corrected k-space data 102 is received. In step 502, the sparse k-space regions 104 are identified within the motion-corrected k-space data 102. In step 504, the alternative magnetic resonance image 300 is received. In step 506, the synthetic magnetic resonance image 302 is received in response to inputting the alternative magnetic resonance image 300 into the magnetic resonance modality conversion neural network 312. In step 508, the synthetic k-space data 304 is generated from the synthetic magnetic resonance image 302. In step 510, the augmented magnetic resonance image 310 is reconstructed using the motion-corrected k-space data 102 such that the sparse k-space regions 104 of the motion-corrected k-space data 102 are augmented with the synthetic k-space data 304.

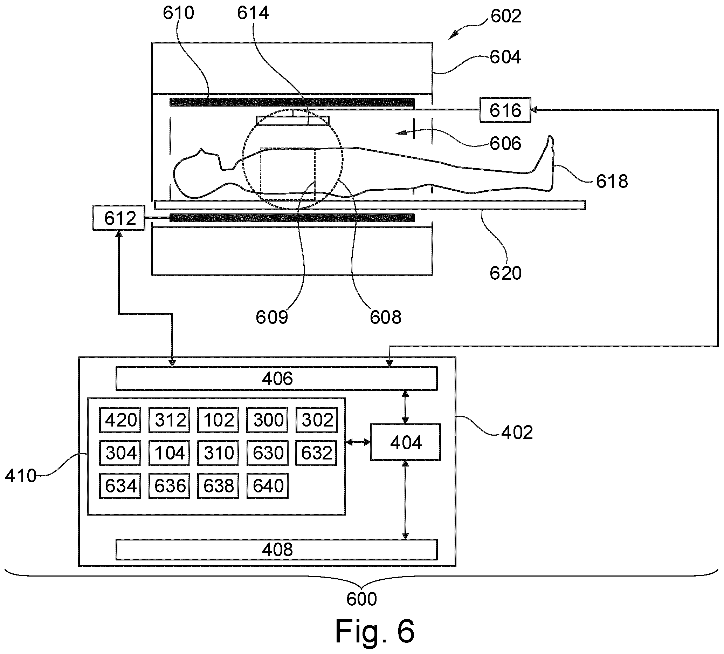

Fig. 6 illustrates a further example of a medical system 600. The medical system 600 depicted in Fig. 6 is similar to that depicted in Fig. 4 except that it additionally comprises a magnetic resonance imaging system 602. The magnetic resonance imaging system 602 comprises a magnet 604. The magnet 604 is a superconducting cylindrical type magnet with a bore 606 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 606 of the cylindrical magnet 604 there is an imaging zone 608 where the magnetic field is strong and uniform enough to perform magnetic resonance imaging. A field of view 609 is shown within the imaging zone 608. The measure k-space data 100 is acquired for the field of view 609. The region of interest could be identical with the field of view 609 or it could be a sub volume of the field of view 609. A subject 618 is shown as being supported by a subject support 620 such that at least a portion of the subject 618 is within the imaging zone 608 and the field of view 609.

Within the bore 606 of the magnet there is also a set of magnetic field gradient coils 610 which is used for the acquisition of the measured k-space data 100 to spatially encode magnetic spins within the imaging zone 608 of the magnet 604. The magnetic field gradient coils 610 are connected to a magnetic field gradient coil power supply 612. The magnetic field gradient coils 610 are intended to be representative. Typically, magnetic field gradient coils 610 contain three separate sets of coils for spatial encoding in three orthogonal spatial directions. A magnetic field gradient power supply supplies current to the magnetic field gradient coils. The current supplied to the magnetic field gradient coils 610 is controlled as a function of time and may be ramped or pulsed.

Adjacent to the imaging zone 608 is a radio frequency coil 614 for manipulating the orientations of magnetic spins within the imaging zone 608 and for receiving radio transmissions from spins also within the imaging zone 608. The radio frequency antenna may contain multiple coil elements. The radio frequency antenna may also be referred to as a channel or antenna. The radio frequency coil 614 is connected to a radio frequency transceiver 616. The radio frequency coil 614 and radio frequency transceiver 616 may be replaced by separate transmit and receive coils and a separate transmitter and receiver. It is understood that the radio frequency coil 614 and the radio frequency transceiver 616 are representative. The radio frequency coil 614 is intended to also represent a dedicated transmit antenna and a dedicated receive antenna. Likewise, the transceiver 616 may also represent a separate transmitter and receiver. The radio frequency coil 614 may also have multiple receive/transmit elements and the radio frequency transceiver 616 may have multiple receive/transmit channels. The transceiver 616 and the gradient controller 612 are shown as being connected to the hardware interface 406 of the computer system 402.

The memory 410 is further shown as containing first pulse sequence commands 630 and second pulse sequence commands 632. The first pulse sequence commands 630 are configured to acquire measured k-space data 100 from the field of view 609 such that it is weighted with the first tissue contrast modality. The memory 410 additionally is shown as storing measured k-space data 100 acquired with the first pulse sequence commands 630. The second pulse sequence commands 632 are configured to acquire alternative k-space data 636 also from the field of view 609 but with a second tissue contrast modality. The measured k-space data 100 is used to generate the motion-corrected k-space data 102 after a motion correction has been performed on the measured k-space data 100. The alternative k-space data 636 is used to reconstruct the alternative magnetic resonance image 300.

The memory 410 is additionally shown as containing a subject motion detection component 638. In this example, the subject motion detection component 638 is a software component which uses the measured k-space data 100 itself to generate the motion data 640. For example, the measured k-space data 100 may have been acquired using a propeller technique and the motion data 640 could be generated from performing self-navigation on the measured k-space data 100. In other examples, the subject motion detection component 638 could be for example a camera or other device which physically measures the position of the subject 618 as different portions of the measured k-space data 100 is acquired.

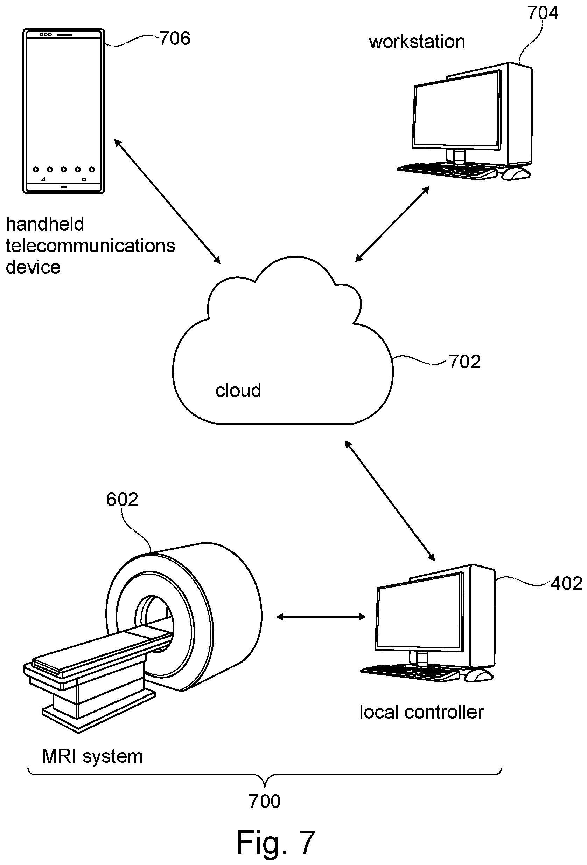

Fig. 7 illustrates a further example of a medical system 700. The medical system 700 comprises a local controller 402, the magnetic resonance imaging system 602 as well as a cloud based computing system 702, and may further comprise any of a radiology workstation 704, and a handheld telecommunications device 706. The functionality of the computer 402 in Figs. 4 and 6 may be distributed amongst the various devices 402, 702, 704, and 706. The cloud based computing system 702 could be made up of networked servers and/or virtual machines available over the internet or other network.

A typical configuration would be to use the local controller 402 to use the pulse sequence commands 630, 632 to control the magnetic resonance imaging system 602 to acquire the k-space data 100, 636. The reconstruction of the augmented magnetic resonance image 310 may be performed by the cloud based computing system 702. The resulting magnetic resonance image 310 would then be made available to the workstation 704, the handheld telecommunications device 706, and the local controller 402.

It is understood that one or more of the aforementioned examples or embodiments of the invention may be combined as long as the combined embodiments are not mutually exclusive.

The term augmenting should be interpreted in the context of the invention as comprising any of: inserting, substituting, combining, supplementing. Combining and supplementing may comprise any form of mathematical, e.g., arithmetical or statistical combination of at least two corresponding data.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An image reconstruction method of reconstructing an augmented magnetic resonance image (310), wherein the method comprises:

   - receiving (500) motion corrected k-space data (102) descriptive of a field of view (609) of a subject (618), wherein the motion corrected k-space data has a first tissue contrast modality;
   - identifying (502) sparse k-space regions (104) within the motion corrected k-space data;
   - receiving (504) an alternative magnetic resonance image (300) descriptive of the field of view of the subject, wherein the alternative magnetic resonance image has a second tissue contrast modality;
   - generating (506) a synthetic magnetic resonance image (302) which has the first tissue contrast modality in response to inputting the alternative magnetic resonance image into a magnetic resonance modality conversion neural network (312);
   - generating (508) synthetic k-space data from the synthetic magnetic resonance image;
   - reconstructing (510) the augmented magnetic resonance image using the motion corrected k-space data such that the sparse k-space regions of the motion-corrected k-space data are augmented with the synthetic k-space data.

2. The image reconstruction method of claim 1, wherein the method further comprises:

   - reconstructing a preliminary magnetic resonance image from the motion corrected k-space data;
   - spatially registering the synthetic magnetic resonance image and the preliminary magnetic resonance image to each other;
   - generating a modified magnetic resonance image by transforming the synthetic magnetic resonance image with any or all of elastic deformations, rigid body translations, and rotations controlled by the registration, wherein the synthetic k-space data is generated from the modified magnetic resonance image.

3. The image reconstruction method of claim 1 or 2, wherein the sparse k-space regions are augmented by inserting the synthetic k-space data from the sparse k-space regions into the measured k-space data.

4. The image reconstruction method of claim 1 or 2, wherein the augmented magnetic resonance image is reconstructed using an optimization, wherein the optimization is preferably configured for performing a parallel imaging reconstruction and/or compressed sensing reconstruction

5. The image reconstruction method of claim 4, wherein the optimization comprises an additional regularization term that incorporates the synthetic k-space data.

6. The image reconstruction method of claim 5, wherein the additional regularization term comprises weighting factors configured for augmenting the sparse k-space regions with the synthetic k-space data.

7. The image reconstruction method of claim 5 or 6, wherein the additional regularization term is configured to use the synthetic magnetic resonance image as a prior step that constrains the optimization.

8. The image reconstruction method of any one of claims 4 through 7, wherein optimization is a joint optimization further configured for performing motion correction, wherein optionally, the sparse k-space regions are repeatedly identified using the joint optimization and the sparse k-space regions of the motion-corrected k-space data augmented with the synthetic k-space data are repeatedly adjusted during the joint optimization.

9. The image reconstruction method of any one of the preceding claims, wherein the sparse k-space regions are determined by searching the measured k-space data for regions below a predetermined k-space sampling density, and wherein the predetermined k-space sampling density preferably decreases with an increasing distance from a central k-space region.

10. The image reconstruction method of any one of the preceding claims, wherein the method further comprises controlling a magnetic resonance imaging system (602), wherein the magnetic resonance imaging system comprises a subject motion detection component (638) configured for measuring motion data (640), wherein the method further comprises:

   - controlling the magnetic resonance imaging system with first pulse sequence commands (630) to acquire measured k-space data (100) descriptive of the field of view of the subject, wherein the measured k-space data is acquired as k-space data portions,
   - acquiring the motion data for the k-space data portions using the subject motion detection component, and
   - constructing the motion corrected k-space data using the measured k-space data and the motion data.

11. The image reconstruction method of claim 10, wherein the first pulse sequence commands are configured to acquire the measured k-space data according to a k-space sampling pattern, wherein the sparse k-space regions are at least partially determined using the k-space sampling pattern and the motion data.

12. The image reconstruction method of claim 10 or 11, wherein the subject motion detection component is any one of the following: a software component configured for deriving the motion data from magnetic resonance navigator data, a software component configured to implement a joint optimization image reconstruction algorithm, a respiratory belt, a camera system, and combinations thereof.

13. A computer program comprising machine executable instructions (420), wherein execution of the machine executable instructions causes a computational system (404) to perform the image reconstruction method of any one of claims 1 through 12.

14. A medical system (400, 600, 700) comprising:

   - a computational system (404) configured to:

      - receive (500) motion corrected k-space data (102) descriptive of a field of view (609) of a subject (618), wherein the motion corrected k-space data has a first tissue contrast modality;
      - identify (502) sparse k-space regions (104) within the motion corrected k-space data;
      - receive (504) an alternative magnetic resonance image (300) descriptive of the field of view of the subject, wherein the alternative magnetic resonance image has a second tissue contrast modality;
      - generate (506) a synthetic magnetic resonance image (302) which has the first tissue contrast modality in response to inputting the alternative magnetic resonance image into a magnetic resonance modality conversion neural network (312);
      - generate (508) synthetic k-space data from the synthetic magnetic resonance image;
      - reconstruct (510) the augmented magnetic resonance image using the motion corrected k-space data such that the sparse k-space regions of the motion-corrected k-space data are augmented with the synthetic k-space data.

15. A data structure comprising:

   - motion corrected k-space data (102) descriptive of a field of view (609) of a subject (618), wherein the motion corrected k-space data has a first tissue contrast modality;
   - synthetic k-space data (304), wherein the synthetic k-space data is descriptive of the field of view of the subject, and wherein the synthetic k-space data has the first tissue contrast modality; and
   - an identification of sparse k-space regions (104) within the motion corrected k-space data.

Fig. 1

100'          102'

Fig. 2

EP 4 707 843 A1

Fig. 3

Fig. 4

500

receive motion corrected k-space data descriptive of a field of view of a subject

502

identify sparse k-space regions within the motion corrected k-space data

504

receive an alternative magnetic resonance image descriptive of the field of view of the subject

506

receive a synthetic magnetic resonance image which has the first tissue contrast modality in response to inputting the alternative magnetic resonance image into a magnetic resonance modality conversion neural network

508

generate synthetic k-space data from the synthetic magnetic resonance image

510

reconstruct the augmented magnetic resonance image using the motion corrected k-space data such that the sparse k-space regions of the motion-corrected k-space data are augmented with the synthetic k-space data

Fig. 5

Fig. 6

EP 4 707 843 A1

706

704
workstation

handheld
telecommunications
device

702
cloud

602

402

MRI system

local controller

700

Fig. 7

Europäisches
Patentamt

European
Patent Office

Office européen
des brevets

**EUROPEAN SEARCH REPORT**

Application Number

EP 24 19 9528

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WON-JOON DO ET AL: "Reconstruction of multicontrast MR images through deep learning", MEDICAL PHYSICS., vol. 47, no. 3, 30 December 2019 (2019-12-30), pages 983-997, XP055742641, US ISSN: 0094-2405, DOI: 10.1002/mp.14006 | 1-8,10, 12-15 | INV. G01R33/56 G01R33/565 G01R33/561 A61B5/055 ADD. G01R33/48 |
| A | * the whole document * | 9,11 | |
| A | Yang Junwei ET AL: "Dual-Domain Multi-Contrast MRI Reconstruction with Synthesis-based Fusion Network", , 1 December 2023 (2023-12-01), pages 1-14, XP093250912, Retrieved from the Internet: URL:https://arxiv.org/pdf/2312.00661 * the whole document * | 2 | |
| A | RIZZUTI GABRIO ET AL: "Joint Retrospective Motion Correction and Reconstruction for Brain MRI With a Reference Contrast", IEEE TRANSACTIONS ON COMPUTATIONAL IMAGING, IEEE, vol. 8, 18 June 2022 (2022-06-18), pages 490-504, XP011912847, ISSN: 2573-0436, DOI: 10.1109/TCI.2022.3183383 [retrieved on 2022-06-20] * the whole document * | 8 | TECHNICAL FIELDS SEARCHED (IPC) G01R A61B |

The present search report has been drawn up for all claims

| Place of search | Date of completion of the search | Examiner |
|---|---|---|
| Munich | 17 February 2025 | Vanhaecke, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YANG, Q.** ; **LI, N.** ; **ZHAO, Z. et al.** MRI Cross-Modality Image-to-Image Translation. *Sci Rep*, 2020, vol. 10, 3753, https://doi.org/10.1038/s41598-020-60520-6